# EUROPEAN PATENT APPLICATION

(11) **EP 1 719 534 A1**
(43) Date of publication of application: **08.11.2006**
(21) Application number: 06113419.3
(22) Date of filing: 03.05.2006
(51) Int. Cl.: A61M 16/00

(54) **Ventilator systems targeting specific patient populations**

(30) Priority: 03.05.2005 US 677613 P
(71) Applicant: China Resorce Group Inc., California Long Beach, CA 90803 (US)
(72) Inventor: GAMBONE, Anthony, Laguna Niguel, CA 92677 (US)
(74) Representative: Dr. Graf & Partner

(57) **Abstract**

A portable ventilator system is loaded with specialized software that addresses specific needs of a targeted patient population. Particularly contemplated target patient populations include patients with chronic obstructive pulmonary diseases (asthma, bronchitis, emphysema, etc.), obstructive sleep apnea, congestive heart failure, neuromuscular paralysis, a high risk of sudden cardiac arrest, and individuals vocationally likely to encounter nuclear, biological, or chemical hazards. Preferred systems include a radio frequency transmitter that sends data from the system to a distal service provider. Contemplated data that can be sent includes location information, diagnostic and intervention information. In another aspect systems can be is integrated with a defibrillator.

## Description

### Field of the Invention.

The field of the invention is breathing assistance devices.

### Background

Breathing is a complicated function, and providing assistance to a victim who is experiencing breathing difficulties is thought to require skilled intervention. The problem is especially difficult to resolve because there are a large number of disease states, each of which requires different intervention protocols. For example, a person having an asthmatic attack requires breathing assistance where the flow rate of the gas is relatively slow, with a slowly ramping flow wave form. However, a person having breathing difficulties due to congestive heart failure would require breathing assistance having a constant positive airway pressure of approximately 10 cm of water.

Although it is conceptually possible to load a single machine with software that could handle all the different scenarios, it is entirely possible that an operator (or the machine itself) would select an incorrect intervention. For patients having a know disease, it can be safer to provide a ventilator that is specifically programmed for that type of disease. Thus, a household may choose to purchase an asthma specific ventilator to accommodate the likely needs of an asthmatic within the household. A neighboring household may choose to purchase a cardiac arrest specific ventilator to accommodate the likely needs of an older member with congestive heart failure. But none of these systems are available in the marketplace, or even contemplated in the prior art.

What is still needed is a portable ventilator system loaded with specialized software that addresses specific needs of a targeted patient population.

### Summary Of The Invention

The present invention provides systems and methods in which a portable ventilator system is loaded with specialized software that addresses specific needs of a targeted patient population. Particularly contemplated target patient populations include patients with chronic obstructive pulmonary diseases (asthma, bronchitis, emphysema, etc.), obstructive sleep apnea, congestive heart failure, neuromuscular paralysis, a high risk of sudden cardiac arrest, and individuals vocationally likely to encounter nuclear, biological, or chemical hazards.

Preferred systems include a radio frequency transmitter that sends data from the system to a distal service provider. Contemplated data that can be sent includes location information, diagnostic and intervention information.

In another aspect systems can be is integrated with a defibrillator.

It should be apparent, however, to those skilled in the art that many more modifications besides those already described are possible without departing from the inventive concepts herein. Moreover, in interpreting the disclosure, all terms should be interpreted in the broadest possible manner consistent with the context. In particular, the terms "comprises" and "comprising" should be interpreted as referring to elements, components, or steps in a non-exclusive manner, indicating that the referenced elements, components, or steps could be present, or utilized, or combined with other elements, components, or steps that are not expressly referenced.

### Brief Description of the Drawing

Fig. 1 is a schematic of interrelationship of different software programs.
Fig. 2 is a schematic of a ventilator according to the present invention, which is wirelessly connected to a GPS satellite, a hospital, and optionally a 911 emergency operations center

### Detailed Description

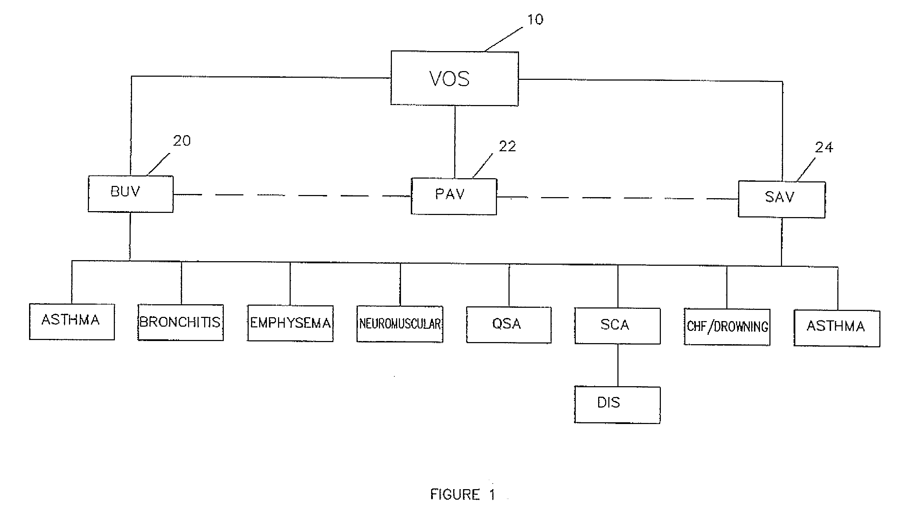

**Figure 1** is a diagram of interrelationship of different software programs. Ventilator Operating System software (**VOS**) 10 runs three software modules, Backup Ventilator (**BUV**) 20, Public Access Ventilation (**PAV**) 22, and Specific Application Ventilation (**SAV**) 24.

BUV 20 operates in the event of system failure. BUV operates independently of all other ventilator software and hardware, which for example can provide an uninterrupted rate of 12 breaths per minute at a tidal volume of 600 ml and a flow rate of 40 liters per minute.

PAV 22 operates a resuscitation mode, aspects of which are described in each of concurrently filed provisional applications, "Neck Positioning Device For Mechanical Ventilator", "Portable Non-Invasive Ventilator With Sensor", and "Ventilator With Rescuer and Victim Guidance" which are incorporated herein by reference in their entirety.

SAV 24 implements algorithms and protocols that are targeted to specific populations. Table 1 below shows currently contemplated differences in the treatment of various different populations using one or a combination of four primary ventilation parameters or modes, pressure, volume, flow and timed ventilation.

**Table 1**

| | |
|---|---|
| asthma | Low flow ventilation with a slowly ramping flow wave form, pressure splinters and aspiratory-only medication delivery |
| bronchitis | Low flow ventilation and pressure support ventilation |
| emphysema | Low flow ventilation and pressure control ventilation |
| neuromuscular | Volume control ventilation and time cycle ventilation |
| obstructive sleep apnea | Pressure assist ventilation |
| sudden cardiac arrest | volume control ventilation and time cycled ventilation |
| congestive heart failure drowning | Pressure control and support ventilation, constant positive airway pressure of approximately 10 cm of water |
| Military NBC (nuclear, biological, or chemical hazards) | Continues flow, pressure support ventilation and time cycle ventilation |

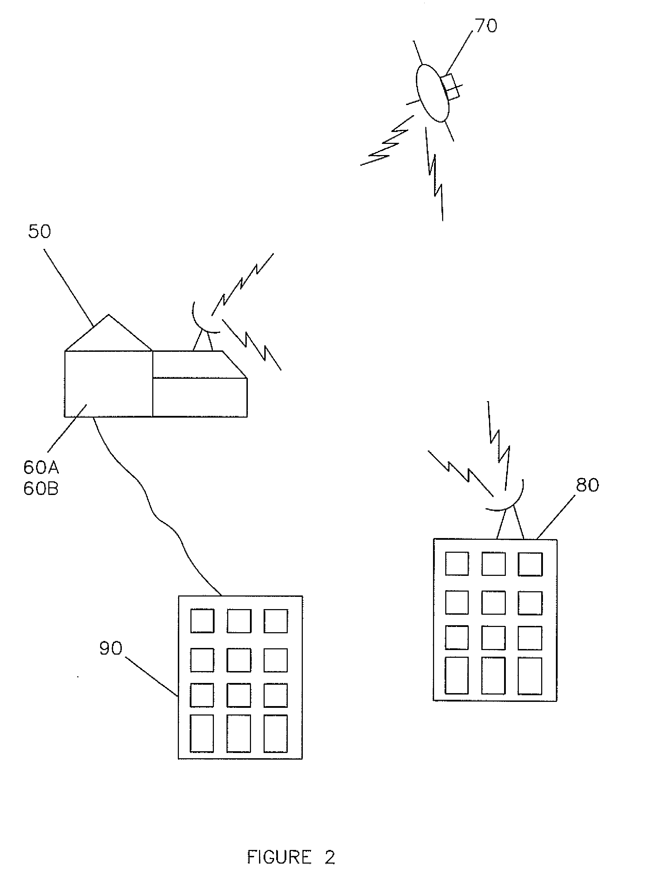

By virtue of different machines addressing different populations, it is contemplated that specific machines would be deployed in specific environments. Thus, for example, a machine loaded with software for asthmatics would be deployed in a grade school, while a platoon of military personnel or a firefighter team might deploy a machine loaded with software for NBC situations. In Figure 2 a house 50 has a combination ventilator 60A / defibrillator 60B, and received signals from a GPS satellite 70. The ventilator 60A / defibrillator 60B sends data to a hospital 80 and possibly independently calls a 911 center 90.

Preferred systems also include a radio frequency transmitter that sends data from the system to a distal service provider. The transmitter can use a police or other reserved band, ordinary cell phone technology, connection through a WAP (wireless access point), or any other suitable technology. Contemplated data to be sent includes location information, diagnostic, and intervention information. Location information can be preloaded by a user, or derived as needed by a GPS (global satellite positioning system), cell phone triangulation, large area network, local area network, and so forth. Contemplated diagnostic information can advantageously include a patient's pulse rate, breathing rate, airway resistance and lung compliance and other lung functions, end tidal CO₂, identity of hazardous agent, ambient temperature, humidity, barometric pressure, and other environmental conditions, as well as operational diagnostics such as battery level, system leaks, component failures etc. Contemplated intervention information can advantageously include duration of intervention, applied pressure, volume and flow of pressurized air.

In another aspect systems can be is integrated with a defibrillator. The ingratiation can exist an any suitable level, from mere juxtaposition within the same carrying case, to inclusion within a common housing, to sharing of power source, display or other user interface, speakers, and so forth.

## Claims

1. A portable ventilator system loaded with specialized software that addresses specific needs of a targeted patient population.

2. The system of claim 1 wherein the target patient population comprises patients with chronic obstructive pulmonary diseases.

3. The system of claim 2 wherein the chronic obstructive pulmonary diseases comprises asthma.

4. The system of claim 2 wherein the chronic obstructive pulmonary diseases comprises bronchitis or emphysema.

5. The system of claim 1 wherein the target patient population comprises patients with obstructive sleep apnea.

6. The system of claim 1 wherein the target patient population comprises patients with congestive heart failure.

7. The system of claim 1 wherein the target patient population comprises patients with a neuromuscular paralysis.

8. The system of claim 1 wherein the target patient population comprises individuals vocationally likely to encounter nuclear, biological, or chemical hazards.

9. The system of claim 1 further comprising a radio frequency transmitter that sends data from the system to a distal service provider.

10. The system of claim 9 wherein the date comprises location information.

11. The system of claim 10 wherein the date comprises diagnostic information derived from analysis of breathing of a victim member of the target population.

12. The system of claim 11 wherein the date comprises intervention information derived from analysis of breathing of a victim member of the target population.

13. The system of claim 1 wherein the target patient population comprises patients with a high risk of sudden cardiac arrest.

14. The system of claim 13 wherein the system is integrated with a defibrillator.
